# EUROPEAN PATENT SPECIFICATION

(11) **EP 4 441 021 B1**
(45) Date of publication and mention of the grant of the patent: **28.01.2026**
(21) Application number: 22829721.4
(22) Date of filing: 01.12.2022
(51) Int. Cl.: C07C 29/80, C07C 31/20, C07C 29/88

(54) **1,3-BUTANEDIOL PURIFIED FROM A MIXTURE OF DIOLS**
AUS EINER MISCHUNG VON DIOLEN GEREINIGTES 1,3-BUTANDIOL
1,3-BUTANEDIOL PURIFIÉ À PARTIR D'UN MÉLANGE DE DIOLS

(30) Priority: 02.12.2021 IT 202100030572
(43) Date of publication of application: 09.10.2024
(73) Proprietor: Novamont S.p.A., 28100 Novara (IT)
(72) Inventor: COTTI COMETTINI, Marco, 13862 Brusnengo (BI) (IT); ALBERTI, Virginia, 18038 Sanremo (IM) (IT)
(74) Representative: Zanoli, Enrico
(86) International application number: PCT/EP2022/084013
(87) International publication number: WO 2023/099650

(56) References cited:
- EP-B1- 0 601 571
- WO-A2-2021/245228
- US-A1- 2016 031 778
- US-A1- 2021 101 855

## Description

The present invention relates to a process for the simultaneous purification of 1,3-butanediol and 1,4-butanediol from a mixture thereof and a composition that simultaneously achieves a high recovery of 1,4-BDO and an increase in the purity of 1,3-BG through this process.

1,3-butanediol (generally known as BG, 1,3-BG, 1,3-BDO or 1,3-butylene glycol) is a four-carbon-atom diol with two stereoisomers: R-1,3-BDO and S-1,3-BDO. The racemic mixture is commonly used in many industrial processes, for example as an organic solvent for food flavouring agents or as a reagent for the production of polyurethane resins and polyesters. Due to its low toxicity and high tolerability, it is also increasingly being used in the cosmetics industry in personal care products, for example in the formulation of hair and bath products, eye and face make-up, perfumes, personal cleansing, shaving and skin care products. Optically active 1,3-BDO is also a widely used component for antibiotics, pheromones, fragrances and insecticides.

1,4-butanediol (generally known as 1,4-BDO, 1,4-BD or 1,4-butylene glycol) is widely used as a monomer for the production of various types of products such as, for example, polyesters of the diacid-diol type or as an intermediate for the synthesis of compounds such as gamma-butyrolactone and tetrahydrofuran. On account of their mechanical and processing properties, polyesters comprising repeating units derived from a dicarboxylic acid and a diol are now widely used in all fields in which thermoplastic polymer materials such as films, moulded and blow-moulded articles and fibres are used. It is also preferable that polyesters obtained in this way are biodegradable, in particular according to EN 13432.

The chemical production of short-chain C₂-C₄ diols from fossil resources has been developed and optimised for decades. 1,3-BG is conventionally produced by a chemical process involving the hydration of acetylene to form acetaldehyde, which is subsequently converted to 3-hydroxybutyraldehyde and reduced to form 1,3-BG. The raceme form is usually obtained in this way.

1,4-BDO can be synthesised via various chemical processes from petrochemical feedstocks: acetylene, via ethynylation with formaldehyde; butadiene, via acetylation or halogenation; propylene, via epoxidation or oxyacetylation; n-butane, via formation of maleic anhydride and its subsequent hydrogenation by various routes.

Due to dwindling fossil resources, fluctuating oil prices and increasing environmental problems the production of C₂-C₄ diols from renewable sources through biological processes has attracted considerable interest.

1,3-BG and 1,4-BDO can in fact be produced via fermentation processes from renewable sources such as carbohydrates, such as sugars and lignocellulosic biomass, or from synthesis gases (CO, CO2 and/or H2), either directly (WO 2015/158716) or via the formation of bio-succinic acid (WO 2011/063055) and its subsequent hydrogenation, or via the formation of polyhydroxyalkanoate (WO 2011/100601).

Patent application WO 2015/158716 describes a process for the production of 1,4-BDO comprising fermentation in a culture medium by a microorganism having at least one metabolic pathway for the synthesis of 1,4-BDO, in which said culture medium comprises a mixture of glucose and sucrose. Similarly, WO 2010/127319 describes a fermentation process for the production of 1,3-BG from renewable sources.

However, 1,3-BG and 1,4-BDO produced from renewable sources generally contain impurities, including by-products resulting directly from fermentation and degradation processes. These impurities need to be removed to ensure that 1,3-BG and 1,4-BDO can be used, for example, in the cosmetics sector and in the synthesis of polyesters of the diacid-diol type, where purities for 1,4-BDO of above 99% for 1,3-BG and above 98%, preferably also above 99%, are required respectively. In these sectors, monomers such as 1,3-BG and 1,4-BDO are in fact all the more sought after, the higher their level of purity.

Furthermore, the above-mentioned fermentation processes generally employ different microorganisms and lead to the production of 1,3-BG and 1,4-BDO characterised by different impurities contents, in both qualitative and quantitative terms.

For example, fermentatively produced 1,4-BDO may contain impurities including 1,4-BDO monoacetate, 2-(4-hydroxybutoxy) tetrahydrofuran, 2-pyrrolidone, tetrahydrofuran, 1,3-propanediol, gamma-butyrolactone, 1,6-hexanediol. Processes for the purification of fermentatively produced 1,4-BDO are for example described in patent applications WO 2019/102030 and WO 2014/152665.

Fermentatively produced 1,3-BDO is usually obtained in a single enantiomeric form, typically the R-form, and may instead contain impurities including 3-hydroxy-butanal, 4-hydroxy-2-butanone, 3-hydroxybutoxy-2-butanone, 1,2-propanediol, 1,3-propanediol, 2,3-butanediol. Processes for purifying fermentatively produced 1,3-BG are for example described in patent application WO 2018/183628.

This requires the identification and implementation of purification processes that meet the requirements of each of the two diols and are adapted to the characteristics of each of the fermentation products. Indeed, the two diols 1,3-BG and 1,4-BDO also have different physical properties and consequently require the implementation of different unitary separation operations, or at least a complex adjustment of the operating conditions of each.

Therefore, although the fermentation processes for the production of the two diols are similar and require essentially the same equipment, a process optimised for the purification of 1,3-BG is generally not suitable for the purification of 1,4-BDO and vice versa.

The process described in patent application IT 102020000013243 permits simultaneous purification of the two diols from their mixture by means of a single process comprising a step of distilling a mixture comprising 1,3-BG and 1,4 BDO and a subsequent step of distilling a fraction comprising 1,3-BG. This allows the two diols to be produced simultaneously, either as a mixture or separately, in the same production plant, with a considerable advantage in terms of time, cost and flexibility, as well as product yields (considering the possible deterioration in the case of prolonged storage), in comparison with conducting separate production and purification runs for 1,3-BG and 1,4-BDO in the same plant, which would instead require significant plant downtime in the transition from one to the other. Said process comprises the removal of solvent (i), a heavy fraction (ii), a light fraction (iii) and a fraction comprising 1,4-BDO (iv) from a mixture comprising 1,3-BG and 1,4-BDO by one or more distillation operations in which at least one of said fractions (iii) and (iv) comprises 1,3-BG. Said fraction comprising 1,3-BG is subsequently subjected to a further distillation.

However, the light fraction (iii) and/or the fraction (iv) comprising 1,3-butanediol obtained in step (a) of this process include impurities, such as by-products resulting from fermentation and degradation processes or formed during distillation operations, which may form azeotropes with the two diols, hindering their purification to the required levels.

In particular it has been observed that certain impurities of 1,4-BDO form azeotropes with 1,3-BG; for example, it has been shown that 1,4-BDO monoacetate (hereafter butanediol monoacetate), one of the typical impurities of 1,4-BDO, forms an azeotrope with 1,3-butanediol. The presence of these impurities means that the desired levels of purity for 1,3-BG cannot be achieved by simple distillation.

Instead, a process has now been identified that improves the separation of 1,3-BG and 1,4-BDO from other compounds present in a mixture thereof that may have physical properties similar to 1,3-BG and 1,4-BDO, including a higher boiling point than water (> 100 °C), solubility in water, or both properties, by introducing a step of the hydrolysis of a distilled fraction that includes 1,3-BG. Besides facilitating purification, this process has the advantage of maximising product recovery yields.

The present invention therefore relates to a process for separating 1,3-butanediol and 1,4-butanediol from a mixture thereof, comprising the steps of:
(a) removing from that mixture, by one or more distillation operations,
   i. the solvent
   ii. a heavy fraction
   iii. a light fraction
   iv. a fraction including 1,4-BDO,
   in which at least one of said fractions (iii) and (iv) comprises 1,3-butanediol and an amount of 1,4-butanediol monoacetate greater than 1 ppm, preferably greater than 100 ppm, and less than 10%, preferably less than 9%, by weight of the composition;
(b) subjecting at least one of the said fractions (iii) and (iv) comprising 1,3-butanediol and 1,4-butanediol monoacetate to at least one hydrolysis reaction;
(c) subjecting at least one of said fractions (iii) and (iv) hydrolysed in step (b) to distillation.

The present invention also relates to a 1,3-butanediol composition comprising butanediol monoacetate in amounts greater than 1 ppm, preferably greater than 100 ppm, and less than 10%, preferably less than 9%, by weight of the composition.

The composition of 1,3-BG obtained at the end of step (c) in the process according to the invention has a concentration of said 1,3-BG that advantageously exceeds 99% by weight and even more advantageously exceeds 99.5% by weight. According to one aspect, the 1,3-BG obtained is a raceme. According to an alternative aspect, the 1,3-BG obtained has a higher content of R enantiomer than S enantiomer.

The composition of 1,4-BDO obtained in fraction (iv) in step (a) or at the end of step (c) advantageously has a concentration of said 1,4-BDO above 98% w/w, more preferably above 99% w/w, even more preferably above 99.5% w/w.

The mixture comprising 1,3-BG and 1,4-BDO fed to this process is obtained by preliminary mixing of the products from the synthesis of 1,3-butanediol and the products from the synthesis of 1,4-butanediol.

The number of distillation operations and the number of columns for each operation in each process step of the invention is not particularly limiting.

The process according to the invention will be described in more detail below.

Step (a) can be conducted by appropriately dimensioning the distillation system to efficiently separate mixtures with different ratios of 1,3-BG to 1,4-BDO. Advantageously, the mixture fed to step (a) in the process according to the invention comprises 1,3-BG and 1,4-BDO having 1,3-BG/1,4-BDO ratios by weight of above 1:99, preferably above 1:33, more preferably above 1:24, more preferably above 1:15, even more preferably above 1:10 and less than 99:1, more preferably less than 3:1, more preferably less than 1:1, more preferably less than 1:2, even more preferably less than 1:5.

Prior to step (a), the process comprises a preliminary step of mixing the synthesis products of 1,3-BG and 1,4-BDO, both comprising reaction by-products and/or impurities and optionally a solvent, in addition to said 1,3-BG and 1,4-BDO. Part of said by-products and/or impurities and/or solvent are advantageously separated from the mixture of 1,3-BG and 1,4-BDO prior to step (a) in the process.

According to a preferred embodiment of the invention, the mixture comprising 1,3-BG and 1,4-BDO subjected to step (a) in the process according to the invention results from the production of 1,3-BG and 1,4-BDO by fermentation. Such fermentative production may take place in separate fermenters, simultaneously or at different times, resulting in the formation of a crude fermentation mixture; preferably the production of the two diols takes place simultaneously in separate fermenters.

According to a preferred aspect, before step (a) the process therefore includes a step of mixing the broths (as the crude mixtures are called) resulting from the fermentative production of 1,3-BG and 1,4-BDO, yielding a crude mixture comprising 1,3-BG and 1,4-BDO.

In this case the process preferably also optionally comprises, before step (a) and advantageously after preliminary mixing step 1), a step of separating one or more fractions containing by-products and impurities from the crude mixture comprising 1,3-BG and 1,4-BDO.

According to a preferred embodiment, the process for separating 1,3-BG and 1,4-BDO from their mixture is therefore preceded by the steps of:
1) mixing the broths from the fermentative production of 1,3-BG and 1,4-BDO, resulting in a crude mixture comprising 1,3-BG and 1,4-BDO,
2) separating one or more fractions comprising solvent, by-products and/or impurities from said crude mixture, resulting in a mixture comprising 1,3-BG and 1,4-BDO.

It is particularly advantageous to feed a homogeneous mixture of the two diols to the above-mentioned step 2) in the process according to the invention.

The production of 1,4-BDO from renewable sources by fermentation is performed, for example, according to the process described in WO 2015/158716.

In WO 2015/158716, the fermentation broth comprising 1,4-BDO from a renewable source and water is generally obtained by a process of fermentation from a culture medium containing at least one sugar, preferably glucose, and optionally one or more sugars other than glucose, in the presence of one or more microorganisms having at least one metabolic pathway for the synthesis of 1,4-BDO.

The culture medium may comprise other substances necessary for growth and sustenance of the microorganism during the fermentation phase, such as elements like C, H, O, N, K, S, P, Fe, Ca, Co, Mn, Mg. Typically the culture medium may include one or more components selected from the group consisting of sugars other than glucose, protein hydrolysates, proteins, amino acids, organic acids, vitamins, mineral salts, yeast extracts, and trace elements such as cobalt, calcium and copper. Cobalt, calcium and copper can be dosed into the culture medium, for example, as salts such as cobalt chloride, calcium chloride and copper chloride. Generally, the culture medium comprises at least one sugar, usually glucose, and optionally one or more sugars other than glucose, in concentrations between 10 and 100 g/L. As the microorganism consumes one or more sugars during the fermentation stage of this process, it is generally necessary to reintroduce these sugars into a fermentation reactor. This reintroduction can be carried out continuously or discontinuously, as known to those skilled in the art. The components of the culture medium, including sugars, protein hydrolysates, proteins, amino acids, organic acids, vitamins, mineral salts, yeast extracts and trace elements are typically added in excess of the microorganism's actual needs and are not therefore fully utilised and remain in the broth at the end of fermentation in the form of impurities.

To limit the unused sugars content and thus optimise the economy of the process, the supply of one or more sugars is advantageously interrupted or gradually decreased before the end of fermentation. As regards other possible components of the culture medium, the culture medium generally contains salts, essential minerals, and antifoaming agents. The culture medium may be prepared in any way known to those skilled in the art, for example by mixing all its components together or by pre-mixing all the components with the exclusion of glucose and adding them later, individually or already pre-mixed in turn. It is also possible to use a commercially available culture medium as a starting point and suitably modify its composition at a later stage, for example when bringing the culture medium into contact with the microorganism having at least one metabolic pathway for the synthesis of 1,4-BDO from a renewable source. During fermentation, the mixture consisting of the microorganism and the culture medium comprising one or more sugars is maintained under conditions suitable for exploiting the metabolic pathway for the synthesis of 1,4-BDO from renewable sources. Those skilled in the art are also able to check the progress of the process during fermentation, for example by monitoring one or more parameters and possibly acting on them to bring the process back to conditions suitable for the production of 1,4-BDO.

The production of 1,3-BG from renewable sources by fermentation is, for example, conducted according to the process described in WO 2010/127319.

Fermentation broth comprising 1,3-BG from a renewable source is generally obtained by a process of fermentation from a culture medium known in the art to support the growth of one or more microorganisms, having at least one metabolic pathway for the synthesis of 1,3-BG, under anaerobic or micro-aerobic conditions. Fermentation is carried out in batch, fed-batch or continuous mode. Anaerobic conditions are maintained by first spraying the medium with nitrogen, while micro-aerobic conditions can also be achieved with a small hole for limited aeration. The pH of the medium is typically maintained at an optimal pH by the addition of a base, such as NaOH or other bases, or an acid.

As regards the metabolic pathways for the synthesis of 1,3-BG and 1,4-BDO, these may be present in the microorganism in their natural state, or they may be artificially created, for example by altering, modifying, amplifying, eliminating or limiting metabolic pathways already present in the microorganism, inserting genetic material from one or more other organisms into it, inducing spontaneous genetic mutations, adding chemical compounds during the process that inhibit or stimulate said metabolic pathways, or otherwise exploiting any genetic engineering technique. Microorganisms with metabolic pathways for the synthesis of 1,4-BDO are known to those skilled in the art and are, for example, described in Yim, H. et al, Nature Chemical Biology, Vol. 7, July 2011, p. 445-452 (hereinafter "Yim et al. 2011") and in patent applications WO 2008/115840, WO 2009/023493, WO 2010/030711, WO 2010/071697, WO 2010/141780, WO 2010/141920, WO 2011/031897, WO 2011/047101, WO 2011/066076, WO 2012/177943, AU 2013/3204409, AU 2013/3204038, AU 2013/202623, AU 2013/203176, AU 2013/203177, AU 2013/203342, AU 2013/203440, AU 2013/203480, AU 2013/203163. Microorganisms having metabolic pathways for the synthesis of 1,3-BG are known to those skilled in the art and are, for example, described in Kataoka, N. et al., Biosci. Biotechnol. Biochem. 2014, Vol 78, p. 695-700 and in patent application WO 2010/127319.

The conversion of sugars into 1,3-BG and 1,4-BDO is typically less than 100 per cent because, in addition to the two diols mentioned, intermediates (by-products) from the metabolic pathways used by microorganisms for the production of 1,3-BG and 1,4-BDO are also produced.

At the end of fermentation, the organisms or cells that make up the cell biomass in the fermentation broth can be deactivated or killed. Deactivation of the biomass or killing of the cells may take place by thermal means, in which case it is typically carried out at a temperature of from about 50°C to 80°C, preferably from about 60°C to 70°C, at least at about 60°C, or at least at about 70°C, for a time of from about 1 minute to 10 minutes, preferably from 2 minutes to 5 minutes, from 2 minutes to 3 minutes, at least 2 minutes or at least 2.5 minutes. This deactivation or killing to which the cell biomass is subjected causes residues and cell metabolites to be released into the fermentation broth.

The broths from the fermentative production of 1,3-BG and 1,4-BDO obtained separately are mixed (step 1), resulting in a crude mixture comprising 1,3-BG and 1,4-BDO.

The term 'crude mixture' within the meaning of the present invention means a broth resulting from 1,3-BG and/or 1,4-BDO fermentation processes.

For example, the crude mixture comprising 1,3-BG and 1,4-BDO comprises, with respect to the total weight of said mixture, approximately 1% to 20% by weight of the sum of 1,3-BG and 1,4-BDO and 80% to 99% by weight of the whole of water and one or more impurities (for example cells, salts, proteins, unconverted sugars, among those mentioned above) derived from fermentation processes.

According to a preferred aspect, the crude mixture comprises, with respect to the total weight of said mixture, approximately 5% to 18% by weight of the sum of 1,3-BG and 1,4-BDO and 82% to 95% by weight of the sum of water and one or more impurities derived from a fermentation process.

According to a more preferred aspect, the crude mixture comprises, with respect to the total weight of said mixture, approximately 10 to 15 per cent by weight of the sum of 1,3-BG and 1,4-BDO and 85 to 90 per cent by weight of the sum of water and one or more impurities derived from a fermentation process.

In the crude mixture, the ratios of 1,3-BG and 1,4-BDO are advantageously determined according to the ratio of the quantities produced by the respective fermentation processes.

The impurities present in the raw mixture include, for example, one or more of the following items: one or more microorganisms, cell residues, any unreacted sugars, by-products, mineral salts, metabolites and components of the culture medium not assimilated or metabolised by these microorganisms.

In order to remove one or more of the impurities described above, in the optional separation step (step 2) in the process according to the preferred embodiment of the invention described above, the raw mixture undergoes one or more treatments chosen from decantation, centrifugation, filtration, microfiltration, nanofiltration, ultrafiltration, ion exchange, osmosis, other suitable solid/liquid separation techniques and combinations thereof.

For example, this raw mixture may be first centrifuged and then filtered, micro-filtered, nano-filtered, treated with ion exchange resins and finally osmoticised.

According to one aspect of the invention, optional separation step 2) comprises one or more passes over one or more ion exchange resins.

Said optional separation step 2) preferably comprises one or more passes over cation exchange resins until a pH between 4 and 2 is obtained at the outlet, and one or more passes over anion exchange resins until a pH between 8 and 11 is obtained at the outlet, as described in patent application WO 2019/102030.

This cation exchange resin is generally selected from the group consisting of resins derived from strong acids (for example sulfonate groups) or weak acids (for example carboxylate groups). The cation exchange resin preferably contains functional groups selected from sulfonate groups. Non-limiting examples of cationic ion exchange resins that are useful in the process according to the invention include, for example, the resin commercially available under the brand name DOWEX^{®} 88 or DOWEX^{®} 88 MB.

Such an anion exchange resin is generally selected from the group consisting of resins derived from strong bases (for example quaternary amine groups) or weak bases (for example tertiary amine groups). The anion exchange resin preferably contains functional groups selected from quaternary amine groups. Non-limiting examples of anion exchange resins that are useful in the process according to the invention include, for example, the resin commercially available under the brand name DOWEX^{®} 22.

The order of the passes over the cationic and anionic exchange resins is not particularly limiting. One or more passes over the cation exchange resins may precede or succeed one or more passes over the anion exchange resins. Preferably, one or more passes over the cation exchange resins precede one or more passes over the anion exchange resins.

Among the operations in optional step 2), operations may be performed to change the solvent content (for example water) of the crude mixture or the intermediate fractions comprising 1,3-BG and 1,4-BDO. Such operations are chosen, for example, from evaporation and reverse osmosis.

A mixture comprising 1,3-BG and 1,4-BDO, optionally obtained according to steps 1) and 2) described above, comprising mainly 1,3-BG, 1,4-BDO and water, is fed to process step (a) according to the present invention. In this, the ratios of 1,3-BG and 1,4-BDO are advantageously set according to the ratio of the quantities produced by the respective fermentation processes. Advantageously said mixture comprises, with respect to its total weight, about 50% to 95% by weight of the sum of 1,3-BG and 1,4-BDO and 5% to 50% by weight of water and one or more impurities derived from the fermentation processes.

In some embodiments, the mixture comprises, in relation to its total weight, approximately 70 to 90 per cent by weight of the sum of 1,3-BG and 1,4-BDO and 10 to 30 per cent by weight of water and one or more impurities derived from a fermentation process.

In some embodiments, the mixture comprises, in relation to its total weight, approximately 75% to 85% by weight of the sum of 1,3-BG and 1,4-BDO and 15% to 25% by weight of water and one or more impurities derived from a fermentation process.

These impurities are, for example, selected from the group consisting of: one or more microorganisms, cell residues, any unreacted sugars, by-products, mineral salts, metabolites and any components of the culture medium not assimilated or metabolised by said microorganism.

Each of the distillation operations in steps a) and c) according to the present invention may be conducted independently by employing different types and configurations of distillation columns. For example, the distillation columns of the process may comprise random-fill, structured-fill, flat-fill, random- and structured-fill, random-fill and flat-fill, or structured-fill and flat-fill sections. Structured-fill columns are preferred.

Each of the distillation operations may be conducted using a single column or train of single columns, or through more integrated configurations that allow more than two streams to be obtained from each column, for example with lateral product extraction or the insertion of vertical baffles to minimise the number of columns and ancillary equipment.

Distillation operations according to this process are preferably carried out by reducing or minimising the exposure of compounds to high temperatures. Both the products and the impurities in them may suffer thermal or chemical degradation caused by heating during distillation. Operation of the distillation columns at reduced pressure (lower than atmospheric pressure) or vacuum is preferable as this lowers the boiling point of the mixture in the distillation column and allows the distillation column to be operated at lower temperatures. A common vacuum system can be used with some or all of the distillation columns to achieve reduced pressure, or each column can have its own vacuum system.

The pressure in a distillation column can be measured at the top or condenser, at the bottom or base, or anywhere in between. The different distillation columns in the process according to the invention can operate at different pressures.

It is possible to adjust the operating conditions in each process step to the type of column(s) used.

The distillation operations in step (a) of the process according to the invention allow solvent (i), a fraction comprising heavy compounds (ii, called the heavy fraction), a fraction comprising light compounds (iii, called the light fraction) and a fraction comprising 1,4-BDO (iv) to be removed.

These components (i)-(iv) may be removed through one or more distillation operations.

Preferably the solvent (i) is water.

During the fermentation process for the production of 1,3-BG and 1,4-BDO, intermediates in the metabolic pathways used by the microorganisms (referred to as by-products) are also produced, and these remain in the broth at the end of fermentation along with other organic compounds such as sugars, protein hydrolysates, proteins, amino acids, organic acids and yeast extracts (which are typically added in excess of the micro-organism's actual needs and are therefore not fully utilised) and may undergo degradation processes. These organic compounds include 'heavy' and 'light' compounds.

'Heavy' refers to compounds with a higher boiling point than 1,4-BDO. Examples of heavy compounds are organic compounds that may have undergone a degradation process, 2-pyrrolidone and 1,6-hexanediol.

'Light' refers to compounds with a lower boiling point than 1,3-BG. Examples of light compounds are 3-hydroxy-butanal, 4-hydroxy-2-butanone, 3-hydroxybutoxy-2-butanone, 1,2-propanediol, 2,3-butanediol and gamma-butyrolactone.

In this application the operating pressures of the distillation columns are measured in absolute millibars (mbar). 1 mbar corresponds to 100 Pascal.

In step (a) of the process according to the invention, according to a first aspect the solvent (i) and optionally traces of light compounds are removed from a mixture comprising, with respect to its total weight, 60-97% by weight, preferably 80-90% by weight of the sum of 1,3-BG and 1,4-BDO, with 1,3-BG/1,4-BDO ratios by weight above 1:99, preferably above 1:33, more preferably above 1:24, more preferably above 1:15, even more preferably above 1:10 and less than 99:1, more preferably less than 3:1, more preferably less than 1:1, more preferably less than 1:2, even more preferably less than 1:5 and from 3% to 40% by weight, preferably from 10 to 20% by weight, of solvent (i), through a distillation operation at a temperature from 20 to 170°C and a head pressure from 10 to 200 mbar, preferably from 80 to 120 mbar. The solvent (i) and optionally traces of light compounds (iii) are separated from the head. The mixture extracted from the bottom preferably comprises an amount of water less than 2000 ppm, more preferably less than 500 ppm and even more preferably less than 200 ppm.

According to another aspect, in step (a) the heavy fraction (ii) is removed from a mixture comprising 1,3-BG and 1,4-BDO with 1,3-BG/1,4-BDO ratios by weight above 1:99, preferably above 1:33, more preferably above 1:24, more preferably above 1:15, even more preferably above 1:10 and less than 99:1, more preferably less than 3:1, more preferably less than 1:1, more preferably less than 1:2, even more preferably less than 1:5 and with an amount of water preferably less than 2000 ppm, more preferably less than 500 ppm and even more preferably less than 200 ppm, through a distillation operation at a temperature from 100 to 170°C, and a head pressure from 10 to 70 mbar, preferably from 20 to 50 mbar. This operation allows the heavy fraction (ii), which comprises at least 50% by weight of heavy compounds and may comprise up to 50% by weight of 1,4-BDO, to be removed from the bottom. This fraction can be treated in a further stage to recover the 1-4 BDO it contains. The column head contains the stream comprising 1,3-BG and 1,4-BDO purified from the heavy fraction (ii).

According to a further aspect, in step (a) the light fraction (iii) is removed from a mixture comprising 1,3-BG and 1,4-BDO in 1,3-BG/1,4-BDO ratios weight above 1:99, preferably above 1:33, more preferably above 1:24, more preferably above 1:15, even more preferably above 1:10 and less than 99:1, preferably less than 3:1, more preferably less than 1:1, more preferably less than 1:2, even more preferably less than 1:5, advantageously through a distillation operation at a temperature from 100 to 170°C, and a head pressure from 10 to 70 mbar, preferably from 20 to 50 mbar. This operation enables removal of the light fraction (iii), which contains at least 1% by weight of impurities and may comprise up to 99% by weight of 1,3-BG. Said light fraction (iii) preferably comprises from 2 to 50% by weight of light compounds and from 50 to 98% by weight of 1,3-BG. The bottom of the column contains mainly purified 1,4-BDO (iv), of purity >95%, preferably >98%, more preferably > 99% and even more preferably > 99.5% by weight. The distillation column may be suitably equipped with a side extraction to allow purging of any fractions with boiling points between those of 1,4-BDO and 1,3-BG.

In step (c) of the process according to the invention, any light fraction (iii) comprising 1,3-BG is advantageously subjected to distillation at a temperature from 20 to 170°C, and a head pressure from 10 to 100 mbar, preferably from 30 to 60 mbar. This allows a stream with a 1,3-BG composition of more than 95%, preferably more than 99% and even more preferably more than 99.5% by weight, to be obtained from the bottom. The distillation head consists mainly of light compounds and can be further treated to increase the recovery of 1,3-BG.

Alternatively, in step (a) the light fraction (iii) is removed from a mixture comprising 1,3-BG and 1,4-BDO in 1,3-BG/1,4-BDO ratios by weight above 1:99, preferably above 1:33, more preferably above 1:24, more preferably above 1:15, even more preferably above 1:10 and less than 99:1, preferably less than 3:1, more preferably less than 1:1, more preferably less than 1:2, even more preferably less than 1:5, advantageously through a distillation operation at a temperature from 100 to 170°C, and a head pressure from 10 to 100 mbar, preferably from 20 to 50 mbar. This operation removes the light fraction (iii), which can comprise up to 95% by weight of 1,3-BG. The bottom of the column comprises 1,3-BG and 1,4-BDO.

In step (c) of the process according to the invention, the fraction (iv) comprising 1,4-BDO and 1,3-BG advantageously undergoes distillation at a temperature from 100 to 170°C and a head pressure from 10 to 100 mbar, preferably from 20 to 70 mbar. This allows a stream with a 1,4-BDO composition of more than 98%, preferably more than 99% and even more preferably more than 99.5% by weight, to be obtained from the bottom. The distillation head consists mainly of 1,3-BG with a purity of more than 95%, preferably more than 98% and even more preferably more than 99% by weight.

The distillation column may be suitably equipped with a side extraction to allow the purging of any fractions with boiling points between those of 1,4-BDO and 1,3-BG.

In a preferred embodiment, step (a) of the process according to the invention comprises the operations of:
a.1) removing the solvent (i) by initial distillation,
a.2) removing the heavy fraction (ii) by a second distillation,
a.3) removing the light fraction (iii) by a third distillation, resulting in a fraction comprising 1,4-BDO (iv),
in which at least one of said fractions (iii) and (iv) includes 1,3-BG.

According to a preferred aspect, said light fraction (iii) comprises 1,3-BG.

According to an alternative aspect, the fraction comprising 1,4-BDO (iv) includes 1,3-BG.

Said light fraction (iii) and/or said heavy fraction (iv) include impurities, such as by-products resulting from fermentation and degradation processes or formed during the distillation operations in step (a), in the form of, for example, acetals and/or esters.

One such impurity is butanediol monoacetate, which is present in the light fraction (iii) in amounts greater than 1 ppm, preferably greater than 100 ppm and advantageously less than 10%, preferably less than 9% by weight.

The process according to the present invention therefore comprises a step (b) of hydrolysis of fraction (iii) comprising 1,3-BG or fraction (iv) comprising the 1,3-BG separated in step (a). The hydrolysis of fraction (iii) is preferred.

When subjected to hydrolysis, some of the impurities in these fractions comprising 1,3 BG separated in step (a), in the form of, for example, acetals and/or esters, form the relevant aldehydes and/or ketones and alcohols.

Preferably, the aldehydes and/or ketones thus obtained are subsequently reduced to alcohols by treatment with a reducing agent, for example according to the process described in Italian patent application 102020000031979.

The alcohols obtained after hydrolysis of said acetals and/or esters, and/or obtained after reduction of said aldehydes and ketones, consist mainly of 1,3-BG and/or 1,4-BDO. For example, 4-hydroxybutanal and 1,4-BDO are formed from the hydrolysis of 2-(4-hydroxybutoxy)-tetrahydrofuran.

The process according to the present invention is therefore particularly advantageous in that it makes it possible to reduce or even eliminate impurities forming azeotropes with 1,3-BG and 1,4-BDO, such as butanediol monoacetate and 2-(4-hydroxybutoxy)-tetrahydrofuran, while at the same time further increasing recovery yields.

Unexpectedly, it has been observed that by feeding a composition comprising 1,3-butanediol and an amount of butanediol monoacetate greater than 1 ppm, preferably greater than 100 ppm and advantageously less than 10%, preferably less than 9% by weight with respect to the weight of the composition, at step (b) in the present process, a high recovery of 1,4-BDO and an increase in the purity of 1,3-BG are simultaneously obtained. The 1,3-BG composition thus obtained at the end of step (c) in the separation process has a purity of even more than 99% by weight, advantageously more than 99.5% by weight. According to one aspect, the butanediol monoacetate content of the 1,3-BDO composition obtained at the end of step (c) in the process is below 1000 ppm.

It is therefore also an object of the present invention to provide a 1,3-butanediol composition comprising butanediol monoacetate in amounts greater than 1 ppm, preferably greater than 100 ppm and advantageously less than 10%, preferably less than 9% by weight of the composition.

Said composition is advantageously obtained from a mixture of fermentatively synthesised 1,3-butanediol and 1,4-butanediol products after removal of the solvent and a heavy fraction, for example by distillation; preferably said composition is obtained in step (a) of the process for the separation of 1,3-butanediol and 1,4-butanediol from a mixture thereof described above. The hydrolysis reaction in the process according to the invention may be conducted according to techniques known to those skilled in the art, for example with water only, catalysed by acids, bases or the addition of one or more enzymes.

In the case of hydrolysis with water alone, the reaction is advantageously conducted at high temperatures and/or pressures. Acid-catalysed hydrolysis and base-catalysed hydrolysis are advantageously conducted for example with the addition of acids and bases respectively, or via ion exchange resins.

The added acid is preferably a strong mineral acid, which can be chosen from, for example, orthophosphoric acid, sulfuric acid and hydrochloric acid.

The added base is preferably a strong base, which can be chosen from for example NaOH, LiOH, KOH and mixtures thereof. Preferably the added base is NaOH.

According to one aspect of the present embodiment, at least one base-catalysed hydrolysis is performed. According to another aspect of the present embodiment, at least one acid-catalysed hydrolysis and at least one base-catalysed hydrolysis are performed.

Preferably, the hydrolysis reaction is conducted in the presence of water in an amount greater than 50% by weight, for example 50 to 99% by weight, preferably 55 to 95% by weight, more preferably 75 to 90% by weight, relative to the total weight of the aqueous solution. Large amounts of water promote the hydrolysis reaction, but all the water must then be removed afterwards, with the associated removal costs.

According to one aspect, hydrolysis is conducted in the presence of an acid or base by keeping the aqueous solution under agitation, preferably at a temperature from 25 to 170°C, preferably from 50 to 100°C, even more preferably from 70 to 95°C, for 1 minute to 240 minutes, preferably from 5 minutes to 120 minutes, even more preferably from 15 minutes to 40 minutes. The aqueous solution obtained at the end of the hydrolysis reaction is advantageously treated to remove water and hydrolysis by-products, for example through one or more treatments selected from: passage over a bed of ion exchange resins, activated carbon, membrane filtration, electrodialysis, flash distillation.

For example, to eliminate ionic species, the aqueous solution obtained after the hydrolysis reaction can be subjected to one or more treatments with ion exchange resins, for example as described above for optional separation step 2). Those skilled in the art will be able to choose the type of resin to be used according to the hydrolysis conditions adopted.

Cation exchange resins, anion exchange resins or a combination thereof are used. Resins are generally chosen from the groups listed above for optional separation step 2).

The aqueous solution obtained at the end of the hydrolysis reaction or, preferably, obtained after passage over the ion exchange resins or between the different passages over said resins, then advantageously undergoes a concentration stage to remove the water present.

This possible concentration step can be performed according to known techniques. For this purpose, one or more operations chosen from reverse osmosis, pervaporation, evaporation, distillation, for example evaporation by thermocompression or multiple effect evaporation may advantageously be used. Preferred techniques are evaporation or in any case techniques that do not require too high temperatures to be reached, as these may deteriorate the product.

According to a preferred embodiment, at least one of fractions (iii) and (iv) comprising 1,3-BG obtained in step (a) undergoes hydrolysis in the presence of a base in step (b), resulting in an aqueous solution of 1,3-BG containing ionic species. According to this embodiment, before step (c) the process comprises the further steps of:
I. optionally subjecting said aqueous solution to one or more treatments with ion exchange resins to remove ionic species from the aqueous 1,3-BG solution, and
II. concentrating the aqueous solution of 1.3-BG.

In this application, the concentrations of the various components are expressed as % by weight.

## Claims

1. Process for separating 1,3-butanediol and 1,4-butanediol from a mixture thereof, comprising the steps of:
a) removing from that mixture, by one or more distillation operations,
i. the solvent
ii. a heavy fraction
iii. a light fraction
iv. a fraction comprising 1,4-butanediol,
in which at least one of said fractions (iii) and (iv) includes 1,3-butanediol and an amount of 1,4-butanediol monoacetate greater than 1 ppm and less than 10% by weight with respect to the weight of the composition;
b) subjecting at least one of said fractions (iii) and (iv) comprising 1,3-butanediol and 1,4-butanediol monoacetate to at least one hydrolysis reaction;
c) subjecting at least one of said fractions (iii) and (iv) hydrolysed in step (b) to distillation.

2. Process according to claim 1, in which these products of synthesis are derived from the production of 1,3-butanediol and 1,4-butanediol by fermentation.

3. Process according to claim 2, comprising, before step (a), a preliminary step of mixing the broths resulting from the fermentative production of 1,3-butanediol and 1,4-butanediol (the crude mixture), a step of separating one or more fractions comprising solvent, by-products and/or impurities, obtaining a mixture comprising 1,3-butanediol and 1,4-butanediol.

4. Process according to one or more of claims 1-3, in which 1,3-butanediol with a purity of more than 99% by weight, preferably more than 99.5% by weight, is obtained from step (c).

5. Process according to one or more of claims 1-4, in which the solvent (i) is water.

6. Process according to one or more of claims 1-5, in which the light fraction (iii) further comprises 3-hydroxy-butanal, 4-hydroxy-2-butanone, 3-hydroxybutoxy-2-butanone, 1,2-propanediol, 2,3-butanediol and/or gamma-butyrolactone.

7. Process according to one or more of claims 1-6, in which step (a) includes the operations of:
(a.1) removing the solvent (i) by initial distillation,
(a.2) removing the heavy fraction (ii) by a second distillation,
(a.3) removing the light fraction (iii) by a third distillation, resulting in a fraction comprising 1,4-butanediol (iv),
in which fraction (iii) comprises 1,3-butanediol.

## Patentansprüche

1. Verfahren zum Abtrennen von 1,3-Butandiol und 1,4-Butandiol von einer Mischung davon, umfassend die Schritte:
a) das Entfernen aus dieser Mischung durch einen oder mehrere Destillationsvorgänge
i. des Lösungsmittels
ii. einer schweren Fraktion
iii. einer leichten Fraktion
iv. einer Fraktion, umfassend 1,4-Butandiol,
wobei mindestens eine der Fraktionen (iii) und (iv) 1,3-Butandiol und eine Menge an 1,4-Butandiolmonoacetat von mehr als 1 ppm und weniger als 10 Gew.-%, bezogen auf das Gewicht der Zusammensetzung, enthält;
b) das Unterziehen mindestens einer der Fraktionen (iii) und (iv), umfassend 1,3-Butandiol und 1,4-Butandiolmonoacetat, mindestens einer Hydrolysereaktion;
c) das Unterziehen mindestens einer der in Schritt (b) hydrolysierten Fraktionen (iii) und (iv) einer Destillation.

2. Verfahren gemäß Anspruch 1, bei dem diese Syntheseprodukte aus der Herstellung von 1,3-Butandiol und 1,4-Butandiol durch Fermentation stammen.

3. Verfahren gemäß Anspruch 2, umfassend vor Schritt (a) einen vorbereitenden Schritt des Mischens von Brühen, die aus der fermentativen Herstellung von 1,3-Butandiol und 1,4-Butandiol resultieren (die Rohmischung), einen Schritt des Abtrennens von einer oder mehreren Fraktionen, umfassend Lösungsmittel, Nebenprodukte und/oder Verunreinigungen, um eine Mischung zu erhalten, die 1,3-Butandiol und 1,4-Butandiol umfasst.

4. Verfahren gemäß einem oder mehreren der Ansprüche 1-3, bei dem 1,3-Butandiol mit einer Reinheit von mehr als 99 Gew.-%, bevorzugt mehr als 99,5 Gew.-%, in Schritt (c) erhalten wird.

5. Verfahren gemäß einem oder mehreren der Ansprüche 1-4, bei dem das Lösungsmittel (i) Wasser ist.

6. Verfahren gemäß einem oder mehreren der Ansprüche 1-5, bei dem die leichte Fraktion (iii) ferner 3-Hydroxybutanal, 4-Hydroxy-2-butanon, 3-Hydroxybutoxy-2-butanon, 1,2-Propandiol, 2,3-Butandiol und/oder Gamma-Butyrolacton umfasst.

7. Verfahren gemäß einem oder mehreren der Ansprüche 1-6, bei dem Schritt (a) die folgenden Vorgänge umfasst:
(a.1) das Entfernen des Lösungsmittels (i) durch eine erste Destillation,
(a.2) das Entfernen der schweren Fraktion (ii) durch eine zweite Destillation,
(a.3) das Entfernen der leichten Fraktion (iii) durch eine dritte Destillation, was eine Fraktion (iv), umfassend 1,4-Butandiol, ergibt,
wobei die Fraktion (iii) 1,3-Butandiol umfasst.

## Revendications

1. Procédé de séparation du 1,3-butanediol et du 1,4-butanediol à partir de leur mélange, comprenant les étapes de :
a) élimination de ce mélange, par une ou plusieurs opérations de distillation,
i. du solvant
ii. d'une fraction lourde
iii. d'une fraction légère
iv. d'une fraction comprenant du 1,4-butanediol,
dans lequel au moins une desdites fractions (iii) et (iv) comprend du 1,3-butanediol et une quantité de monoacétate de 1,4-butanediol supérieure à 1 ppm et inférieure à 10 % en poids, par rapport au poids de la composition ;
b) soumission d'au moins une desdites fractions (iii) et (iv) comprenant du 1,3-butanediol et du monoacétate de 1,4-butanediol à au moins une réaction d'hydrolyse ;
c) soumission d'au moins une desdites fractions (iii) et (iv) hydrolysées à l'étape (b) à une distillation.

2. Procédé selon la revendication 1 dans lequel ces produits de synthèse sont issus de la production de 1,3-butanediol et de 1,4-butanediol par fermentation.

3. Procédé selon la revendication 2, comprenant, avant l'étape (a), une étape préliminaire de mélange des bouillons issus de la production par fermentation du 1,3-butanediol et du 1,4-butanediol (le mélange brut), une étape de séparation d'une ou plusieurs fractions comprenant du solvant, des sous-produits et/ou des impuretés, obtention d'un mélange comprenant du 1,3-butanediol et du 1,4-butanediol.

4. Procédé selon une ou plusieurs des revendications 1 à 3, dans lequel du 1,3-butanediol ayant une pureté supérieure à 99 % en poids, de préférence supérieure à 99,5 % en poids, est obtenu à l'étape (c).

5. Procédé selon une ou plusieurs des revendications 1 à 4, dans lequel le solvant (i) est l'eau.

6. Procédé selon une ou plusieurs des revendications 1 à 5, dans lequel la fraction légère (iii) comprend en outre du 3-hydroxy-butanal, de la 4-hydroxy-2-butanone, de la 3-hydroxybutoxy-2-butanone, du 1,2-propanediol, du 2,3-butanediol et/ou de la gamma-butyrolactone.

7. Procédé selon une ou plusieurs des revendications 1 à 6, dans lequel l'étape (a) comprend les opérations de :
(a.1) élimination du solvant (i) par distillation initiale,
(a.2) élimination de la fraction lourde (ii) par une deuxième distillation,
(a.3) élimination de la fraction légère (iii) par une troisième distillation, conduisant à une fraction comprenant du 1,4-butanediol (iv),
dans lequel la fraction (iii) comprend du 1,3-butanediol.
